# EUROPÄISCHE PATENTSCHRIFT

(11) **EP 2 554 539 B1**
(45) Veröffentlichungstag und Bekanntmachung des Hinweises auf die Patenterteilung: **10.12.2014**
(21) Anmeldenummer: 11176250.6
(22) Anmeldetag: 02.08.2011
(51) Int. Cl.: C07D 209/54

(54) **Verfahren zur Herstellung von gamma-Spirolactamen aus zyklischen ungesättigten Carbonsäuren**
Method for producing gamma-spirolactams from cyclic unsaturated carboxylic acids
Procédé de fabrication de spirolactames gamma à partir d'acides carboniques cycliques insaturés

(43) Veröffentlichungstag der Anmeldung: 06.02.2013
(73) Patentinhaber: Universität Potsdam, 14469 Potsdam (DE)
(72) Erfinder: Linker, Prof. Dr. Torsten, 14469 Potsdam (DE); Krüger, Tobias, 14550 Groß Kreutz (DE)
(74) Vertreter: Gulde & Partner

(56) Entgegenhaltungen:
- WO-A2-2007/127763
- REDDY P A ET AL: JOURNAL OF MEDICINAL CHEMISTRY, AMERICAN CHEMICAL SOCIETY, US, Bd. 39, Nr. 9, 1. Januar 1996 (1996-01-01) , Seiten 1898-1906, XP002099346, ISSN: 0022-2623, DOI: 10.1021/JM9600196

## Beschreibung

Die Erfindung betrifft ein Verfahren zur Herstellung von gamma-Spirolactamen aus zyklischen Carbonsäuren, bevorzugt aus aromatischen Carbonsäuren. Die Verbindungen eignen sich bevorzugt als wertvolle Zwischenprodukte zur Herstellung von Peptidomimetika. Die Erfindung betrifft auch die Verwendung des Verfahrens zur Herstellung entsprechender Peptidomimetika.

Peptidomimetika sind eine chemisch heterogene Gruppe niedermolekularer organischer Verbindungen. Trotz einer Vielzahl an Veränderungen ist meist eine mehr oder minder ausgeprägte strukturelle Ähnlichkeit zu Peptiden zu erkennen. So finden Peptidomimetika in der Pharmakologie als Liganden von Rezeptoren für Peptide und Proteine und als Substratanaloga für Peptidasen Anwendung. Als Rezeptorliganden können sie diese aktivieren (Agonist) oder hemmen (Antagonist).

Gamma-Spirolactam-Derivate (γ-Spirolactam-Derivate) sind von großer Bedeutung in der Chemie und Medizin, denn durch deren zyklische Form kann das Peptidomimetikum in einer Konformation fixiert werden, die für eine Interaktion mit dem Zielmolekül optimal ist. Die γ-Spirolactame stellen als sogenannte Schlüsselprodukte Zwischenprodukte bei der Synthese biologisch nützlicher γ-Spirolactam-Derivate als Peptidomimetika dar (z.B. Rezeptor-Antagonisten oder Inhibitoren, welche in den PCT-Veröffentlichungen WO 2006/031606 A (EP 1 796 678B1) oder WO 2007/127763 A (EP 2 035 379 B1) beschrieben sind). Weitere Synthesen zur Herstellung von γ-Spirolactam-Derivaten als Peptidomimetika sind beschrieben worden. z.B. von X. Xu, X. Chje, S. Gao, J. Wu, X. Bai, Synlett 2005, 1865. Insbesondere Strukturen umfassend einen Cyclohexanring sind von großem Interesse für die pharmazeutische Industrie. Die Synthesen erfordern jedoch mehrere Schritte, umfassen teure und toxische Reagenzien und erzeugen z.T. geringe Ausbeuten. Gemäß EP 1 796 678 B1 werden z.B. vierzehn Stufen benötigt um zu dem Schlüsselderivat zu gelangen, gemäß EP 2 035 379 B1 zehn Stufen.

Ausgehend von der Tatsache, dass es bisher keine effiziente und kostengünstige Synthese für γ-Spirolactame gibt, ist es für eine kommerzielle Verwendung notwendig, Verfahren zu finden, die wenige Stufen umfassen und explosive sowie toxische Reagenzien im Wesentlichen vermeiden.

Die Aufgabe der Erfindung bestand deshalb darin, γ-Spirolactame in einer einfacheren und kostengünstigeren Weise zu produzieren als es bisher beschrieben wurde.

Das o.g. Problem konnte durch Anwendung der Birch-Reduktion gelöst werden und es wird eine einfache Synthese von γ-Spirolactamen der allgemeinen Formel 1 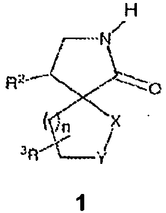 bereitgestellt, worin bedeuten
- R²: H, substituiertes oder unsubstituiertes C₁ bis C₂₀ -Alkyl, C₂ bis C₂₀-Alkenyl, C₂ bis C₂₀-Alkinyl, C₁ bis C₂₀ -OAlkyl, C₂ bis C₂0 -OAlkenyl, C₂ bis C₂₀-OAlkinyl, Halogen, SH, NH₂, CN, NO₂, SO₃, eine Carbonyl- Thiocarbonyl-, Imino-, Carboxy-, C₁-C₂₀-Alkoxycarbonyl- oder Carboxamidgruppe, substituiertes oder unsubstituiertes C₅-C₂₀ Aryl, C₅-C₂₀ Heteroaryl, und
- R³: jeweils ein- oder mehrfach H, substituiertes oder unsubstituiertes C₁ bis C₂₀ -Alkyl, -Alkenyl, -Alkinyl, C₂ bis C₂₀ -OAlkyl, C₂ bis C₂₀ -OAlkenyl, C₂ bis C₂₀-OAlkinyl, Halogen, Hydroxy, SH, NH₂, CN, NO₂, SO₃, eine Carbonyl- Thiocarbonyl-, Imino-, Carboxy-, C₁-C₂₀-Alkoxycarbonyl- oder Carboxamidgruppe, substituiertes oder unsubstituiertes C₅-C₂₀ Aryl, C₅-C₂₀ Heteroaryl, Arylalkyl, C₃-C₈-Cycloalkyl, -Cycloalkylalkyl, wobei Alkenyle und Alkinyle auch an zwei nebeneinander liegenden C-Atomen des Cyclogrundgerüsts der Verbindung 1 gebunden sein können und die Doppel- bzw. Dreifachbindungen mit dem Ring konjugiert sein können,
- X und Y: gleich oder verschieden CH₂, NH, O, S oder gemeinsam CH = CH, sowie
- n=: 1,2,3.

Das Verfahren ist dadurch gekennzeichnet, dass man eine entsprechende zyklische Carbonsäure der allgemeinen Formel 2 mit einem Halogenacetonitril der allgemeinen Formel 3 - HalCHR₂-CN - einer Birch-Reduktion in Gegenwart von Alkalimetallen in flüssigem Ammoniak unterwirft und zu Verbindungen der allgemeinen Formel 4 reduziert. Durch eine sich anschließende katalytische Hydrierung wird das entstandene Nitril 4 zum entsprechenden Amin der allgemeinen Formel 5 reduziert. Durch Erwärmung und Dehydratisierung werden die Zielverbindungen 1 gewonnen.

Die Reaktion ist durch das folgende Schema darstellbar: 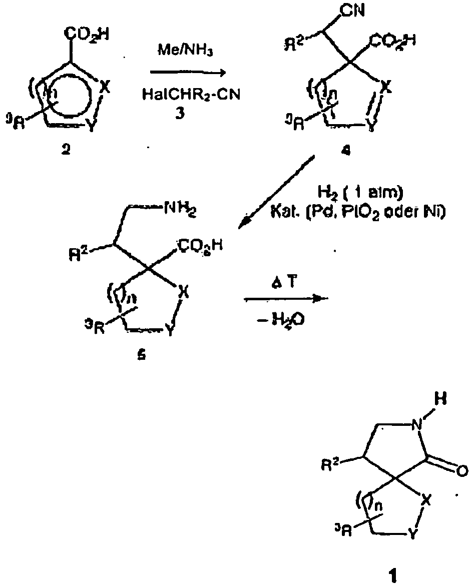 wobei R², R³, X, U und n die obigen Bedeutungen besitzen, Hal für Cl oder Br steht und Me Li, Na oder K bedeutet, und wobei in einem ungesättigten Sechsring NH durch N ersetzt ist.

Die Formulierung substituiert schließt insbesondere Substituenten ein, ausgewählt aus der Gruppe umfassend einfach oder mehrfach jeweils unabhängig voneinander Wasserstoff, Halogen, geradkettiges oder verzweigtes Alkyl mit 1-20 C-Atomen, geradkettiges oder verzweigtes Alkenyl mit 2-20 C-Atomen und einer oder mehreren Doppelbindungen, geradkettiges oder verzweigtes Alkinyl mit 2-20 C-Atomen und einer oder mehreren Dreifachbindungen, gesättigtes, teilweise oder vollständig ungesättigtes Cycloalkyl mit 3-7 C-Atomen, welches mit Alkylgruppen mit 1-6 C-Atomen substituiert sein kann, haben,
wobei die Substituenten paarweise durch Einfach- oder Doppelbindung zu aromatischen oder aliphatischen Ringen miteinander verbunden sein können und wobei ein Kohlenstoffatom oder zwei nicht benachbarte Kohlenstoffatome eines oder mehrerer Substituenten durch Atome und/oder Atomgruppierungen, ausgewählt aus der Gruppe.- O-,- C (O)-,-C (O) O-,-S-, -S (O)-,-SO₂-,-SO₃-,-N=,-N=N-,-NH-,-NR⁴-,-PR⁴-,-P (O) R⁴-,-P (O) R⁴-O-,- O-P (O) R⁴-O-, und -P(R⁴)₂=N- ersetzt sein können, wobei R⁴ nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Aryl oder unsubstituierter oder substituierter Heterocyclus ist, der mit Alkylgruppen mit 1-6 C-Atomen substituiert sein kann.

Es werden besonders bevorzugt mit dem erfindungsgemäßen Verfahren γ-Spirolactam-Verbindungen der Formel 1 hergestellt, in denen
R² Wasserstoff, Halogen, C₁-C₆-Alkyl, Aryl oder Heteroaryl bedeutet.
R³ bedeutet bevorzugt einfach oder mehrfach H, C₁ bis C₆ Alkyl oder C₂ bis C₆ Alkenyle und Alkinyle, die auch an zwei nebeneinander liegenden C-Atomen des Cyclogrundgerüsts der Verbindung 1 gebunden sein können und die Doppel- bzw. Dreifachbindungen mit dem Ring konjugiert sein können, sowie Carbonyl-, Carboxyl-, Carboxamid-, Ester- oder Sulfoxylgruppen oder Aryle und Heteroaryle, die ggf. sämtlich substituiert sein können.
n ist bevorzugt 1 oder 2, besonders bevorzugt 2.

Schlüsselschritt ist die Birch-Reduktion zu der Zwischenstufe 4. Die Durchführung der Birch-Reduktion ist mit Halogenacetonitrilen 3 bisher nicht bekannt. Als Halogenacetonitrile werden im erfindungsgemäßen Verfahren bevorzugt Chlor- oder Bromacetonitrile eingesetzt, wobei R² die oben genannten Bedeutungen besitzt. Besonders bevorzugt wird Chloracetonitril im erfindungsgemäßen Verfahren zur Birch-Reduktion verwendet.

Das Alkalimetall ist ausgewählt aus der Gruppe umfassend Lithium, Natrium und Kalium. In einer bevorzugten Ausführungsvariante des erfindungsgemäßen Verfahrens wird als Alkalimetall Lithium verwendet.

Der Ammoniak wird im Anschluss an die Birch-Reduktion in der Regel verdunstet und der Rückstand kann mit einem geeigneten Lösungsmittel extrahiert werden. Das Extraktionsmittel ist vorzugsweise ausgewählt aus der Gruppe der organischen Lösungsmittel z.B. Halogenkohlenwasserstoffen, Ethern oder Estern.

Im nächsten Schritt wird durch katalytische Hydrierung das Nitril 4 zum Amin 5 reduziert und gleichzeitig der Ring teilweise oder vollständig gesättigt. Katalysatoren können Pd, Pt, PtO₂, Ni oder andere Übergangsmetalle sein. Solche Katalysatoren sind dem Fachmann bekannt. Die katalytische Hydrierung verläuft bevorzugt in einem protischen Lösungsmittel, z.B. einem Alkohol, sowie unter Einsatz von Wasserstoff bei vorzugsweise 1atm und Raumtemperatur (20-25°C). Vorteil dieser Stufe ist der Katalyseprozess und Einsatz von kostengünstigem Wasserstoff bei bevorzugter 1 atm.

In der letzten Stufe wird durch einfaches Erwärmen auf bevorzugte Temperaturen von 80 bis 140°C dehydratisiert (z. B. in Pyridin) und der γ-Lactamring zur Verbindung 1 geschlossen.

Bevorzugt werden substituierte und unsubstituierte zyklische Carbonsäuren oder Heterocarbonsäuren der Formel 2 eingesetzt, in denen n = 1 oder 2 ist.
R³ bedeutet bevorzugt einfach oder mehrfach H, C₁ bis C₆ Alkyl, vorzugsweise Methyl, oder C₂ bis C₆ Alkenyle oder Alkinyle, die auch an zwei nebeneinander liegenden C-Atomen des Cyclogrundgerüsts der Verbindung 1 gebunden sein können und die Doppel- bzw. Dreifachbindungen mit dem Ring konjugiert sein können, sowie Carbonyl-, Carboxyl-, Carboxamid-, Ester- oder Sulfoxylgruppen oder Aryle und Heteroaryle, die ggf. substituiert sein können.

Beispiele geeigneter zyklischer Carbonsäuren oder Heterocarbonsäuren, die ggf. in Form ihrer geschützten Derivate verwendet werden können, sind Furan-2-carbonsäure, Pyrrol-2-carbonsäure, Thiophen-2-carbonsäure, Isoxazolcarbonsäure, Isoxthiazolcarbonsäure, 1 H-Pyrazolcarbonsäure, Benzoesäure, Salicylsäure, 3,4,-Dihydroxybenzoesäure, o-Toluylsäure, m-Toluylsäure, p-Toluylsäure, 1-Naphthoesäure, 2-Naphthoesäure, 9-Anthracoesäure, 2-Pyridincarbonsäure, 3-Pyridincarbonsäure, 4-Pyridincarbonsäure, 2-Chinolincarbonsäure, 3-Chinolincarbonsäure, Isochinolin-1-carbonsäure, oder andere zyklische Carbonsäuren, die ggf. mit weiteren Resten R³ substituiert sein können.

Gemäß einer besonderen Ausführungsform der vorliegenden Erfindung werden bevorzugt γ-Spirolactame der allgemeinen Formel 1 mit Cyclohexanring (n=2) hergestellt. Ausgangscarbonsäuren sind vorzugsweise die folgenden Carbonsäuren und Heterocarbonsäuren:
Benzoesäure, 3,4,-Dihydroxybenzoesäure, Salicylsäure, o-Toluylsäure, m-Toluylsäure, p-Toluylsäure, 1-Naphthoesäure, 2-Naphthoesäure, 9-Anthracoesäure, 2-Pyridincarbonsäure, 3-Pyridincarbonsäure, 4-Pyridincarbonsäure, Pyridazincarbonsäure, 2-Chinolincarbonsäure, 3-Chinolincarbonsäure, Isochinolin-1-carbonsäure.

Besonders bevorzugt werden im erfindungsgemäßen Verfahren Benzoesäure, o-Toluylsäure, m-Toluylsäure, p-Toluylsäure, 1-Naphthoesäure, 2-Naphthoesäure oder 9-Anthracoesäure zur Herstellung von Verbindungen der allgemeinen Formel 1 eingesetzt.

"Alkyl" kann eine verzweigte oder unverzweigte Alkylgruppe sein, die vorzugsweise 1 bis 10 C-Atome, besonders bevorzugt 1 bis 6 C-Atome aufweist, z.B. Methyl, Ethyl, n-Propyl, iso-Propyl, n-Butyl, iso-Butyl, s-Butyl, t-Butyl, n-Pentyl, iso-Pentyl, Neopentyl, t-Pentyl, 1-Methylbutyl, 2-Methylbutyl, 1-Ethylpropyl, 1,2-Dimethylpropyl und n-Hexyl.

"Alkenyl" und "Alkinyl" weisen mindestens eine Doppel- bzw. Dreifachbindung auf. Sie können verzweigt oder unverzweigt sein und weisen vorzugsweise 2 bis 10 C-Atome besonders bevorzugt 2 bis 6 C-Atome, auf. Beispielsweise werden hierfür genannt: Propylen, Butylen, Pentylen.

"Aryl" bezeichnet Phenyl, Naphthyl, Indenyl, Tetrahydronaphthyl, Indanyl, Anthracenyl oder Fluorenyl.

"Heteroaryl" bezeichnet 5- bis 10-gliedrige einzelne oder benzoanellierte aromatische Ringe, die 1 bis 4 Heteroatome unabhängig ausgewählt aus der Gruppe bestehend aus - O-, -S- und -N= enthalten, unter der Voraussetzung, dass die Ringe nicht benachbarte Sauerstoff- und/oder Schwefelatome enthalten.

Unter dem Begriff "Heterocyclus " werden stabile 4-, 5- oder 6-gliedrige heterocyclische Ringsysteme verstanden, die sowohl gesättigt als auch einfach ungesättigt sein können und neben Kohlenstoffatomen ein oder zwei Heteroatome tragen können, die ausgewählt sind aus der Gruppe bestehend aus Stickstoff, Sauerstoff und Schwefel. Dabei können sowohl Stickstoff- als auch Schwefelheteroatome optional oxidiert sein. Die voranstehend genannten Heterocyclen können über ein Kohlenstoffatom oder über ein Stickstoffatom mit dem restlichen Molekül verknüpft sein.

Halogen ist Chlor, Brom, Jod, vorzugsweise Cl.

Der Begriff "Arylalkyl" steht für eine geradkettige oder verzweigte (C1-C6)-Alkylgruppe, die durch eine Arylgruppe, wie sie oben definiert worden ist, substituiert ist;
der Begriff "Cycloalkylalkyl" für eine geradkettige oder verzweigte (C1-C6)-Alkylgruppe, die durch eine oder mehrere (C3-C8)-Cycloalkylgruppen substituiert ist.

Mit dem erfindungsgemäßen Verfahren werden überraschend gute Ausbeuten für γ-Spirolactam-Verbindungen der allgemeinen Formel 1 von 70% und darüber erreicht. Bei den Hydrierungen können Stereozentren mit sehr hoher Selektivität aufgebaut werden.

Die Ausgangsverbindungen sind in der Regel kommerziell verfügbar, was das Verfahren sehr günstig macht. Das Verfahren eignet sich besonders zur Herstellung der als Zwischenprodukte verwendbaren Verbindungen 1 und 4.

Durch zusätzliche Schritte, die dem Fachmann bekannt sind, können die γ-Spirolactam-Verbindungen 1 weiter umgesetzt werden, z.B. zu Verbindungen gemäß EP 1 796 678B1 oder 2 035 379 B1.

So kann die Verbindung 1 nach an sich bekannten Verfahren zu Verbindungen der allgemeinen Formel 1a überführt werden:

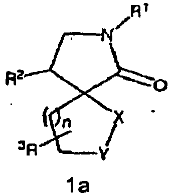

R², R³, X, U und n besitzen die für Verbindung 1 genannten Bedeutungen, R' kann sämtliche der für R³ genannten Bedeutungen besitzen.

Zusammenfassend besteht der Vorteil der Erfindung in einem neuen einfachen Syntheseweg, der auch in großem Maßstab durchführbar ist, weiterhin in der Verwendung von einfachen und kostengünstigen Reagenzien sowie den guten Ausbeuten.

### Ausführungsbeispiel:

Das folgende Schema zeigt die Umsetzung von Benzoesäure zur Zielverbindung 1

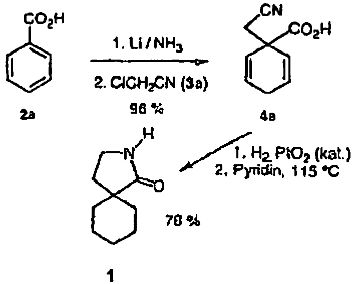

In Analogie werden Gamma-Spirolactame der Toluylsäure und 1-Naphthoesäure gewonnen:

Unter Trockeneis-Acetonkühlung werden 100 mmol Benzoesäure **2a**, Tolylsäure **2b-d** oder Napthoesäure **2e** vorgelegt und unter Rühren 200 mL Ammoniak einkondensiert. Anschließend werden portionsweise 1.39 g (200 mmol) Lithium zugegeben, bis sich die Lösung über gelb nach blau verfärbt und noch 1 h gerührt. Im Anschluss werden 10 mL (160 mmol) Chloracetonitril **3a** zugetropft, bis sich die Lösung komplett entfärbt hat. Über Nacht verdunstet der Ammoniak und der feste Rückstand wird in 70 mL Wasser gelöst, mit 6 M HCl (*unter Eiskühlung*) bis auf pH 2 angesäuert und mit 3 x 50 mL Dichlormethan extrahiert. Die organische Phase wird über Natriumsulfat getrocknet und das Lösemittel am Vakuum entfernt. Der Rückstand wird aus n-Hexan kristallisiert und liefert die Cyclohexadiene **4** als weiße Kristalle.

20 mmol Cyclohexadien **4** werden in 50 mL Methanol gelöst und 50 mg Platinoxid als Katalysator zugesetzt. Dann wird ein mit Wasserstoff gefüllter Ballon aufgesetzt und 5 min Wasserstoff unter **Rühren** durch die Suspension geleitet. Nach Zusatz von 3 mL Salzsäure (37%) wird 24 h bei Raumtemperatur und aufgesetztem Wasserstoffballon gerührt. Der Katalysator wird über Celite abfiltriert, das Lösungsmittel am Rotationsverdampfer abdestilliert und das Rohprodukt im Vakuum getrocknet. Das so erhaltene Rohprodukt wird in 50 mL Pyridin über 3 h am Rückfluss erhitzt. Anschließend wird das Pyridin abdestilliert und das Rohprodukt über Kieselgel 60 säulenchromatographisch (Hexan : Ethylacetat 1:1) gereinigt. Die γ-Spirolactame **1** werden in Form farbloser Kristalle isoliert. Produkte **1b** und **1 c** entstehen in diastereomerenreiner Form!

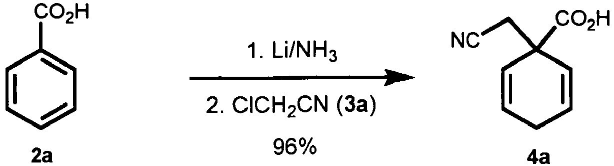

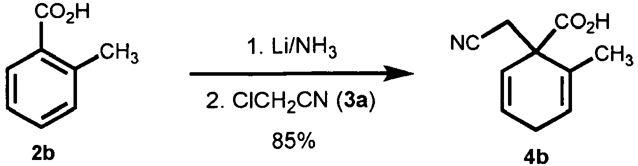

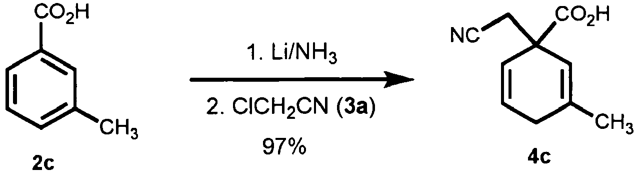

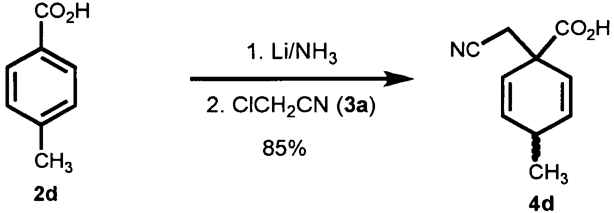

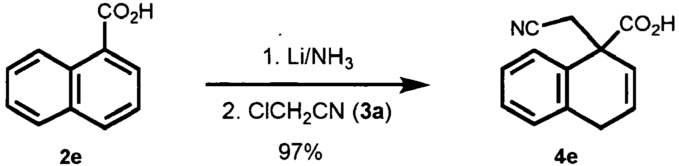

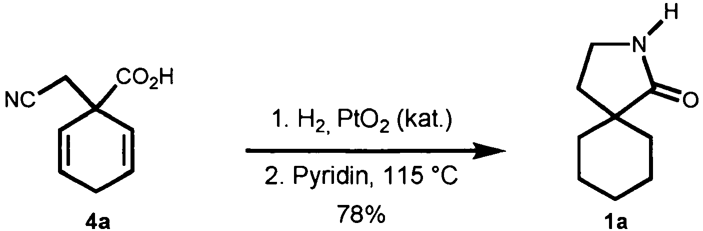

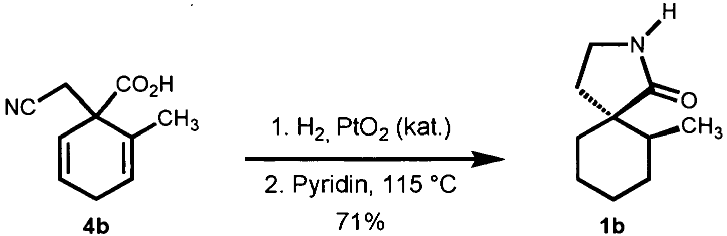

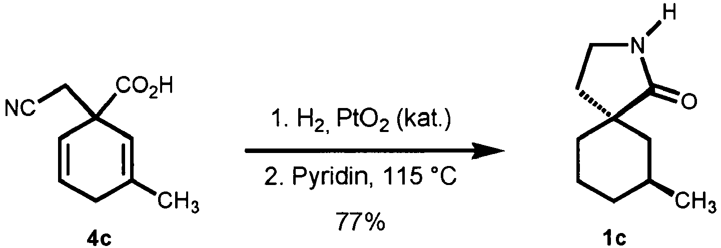

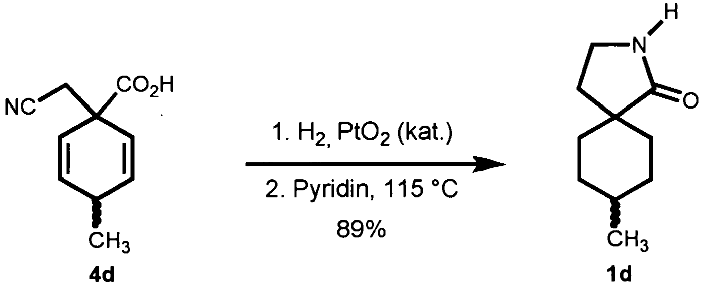

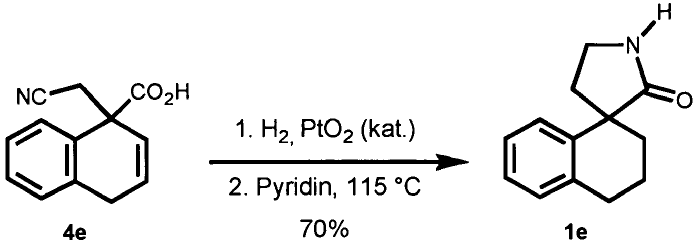

## Patentansprüche

1. Verfahren zur Herstellung von γ-Spirolactamen der allgemeinen Formel I 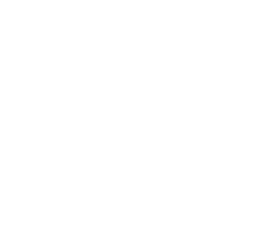 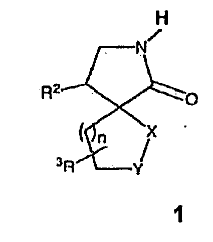 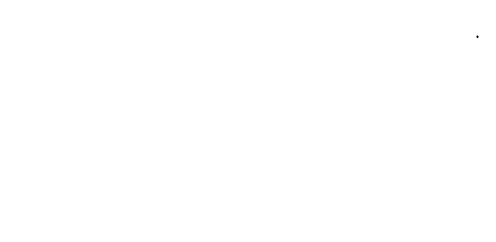 worin bedeuten
R² H, substituiertes oder unsubstituiertes C₁-C₂₀-Alkyl, C₂-C₂₀-Alkenyl, C₂-C₂₀ Alkinyl, C₁-C₂₀-OAlkyl, C₂-C₂₀-OAlkenyl, C₂-C₂₀-OAlkinyl, Halogen, SH, NH₂, CN, NO₂, SO₃, eine Carbonyl- Thiocarbonyl-, Imino-, Carboxy-, C₁-C₂₀-Alkoxycarbonyl- oder Carboxamidgruppe, substituiertes oder unsubstituiertes C₅-C₂₀-Aryl, C₅-C₂₀-Heteroaryl, und
R³ jeweils ein- oder mehrfach H, substituiertes oder unsubstituiertes C₁-C₂₀-Alkyl, C₂-C₂₀-Alkenyl, C₂-C₂₀-Alkinyl, C₁-C₂₀-OAlkyl, C₂-C₂₀-OAlkenyl, C₂-C₂₀-OAlkinyl, C₃-C₈-Cycloalkyl, C₃-C₈-Cycloalkylalkyl_{;} Halogen, Hydroxy, SH, NH₂, CN, NO₂, SO₃, eine Carbonyl- Thiocarbonyl-, Imino-, Carboxy-, C₁-C₂₀-Alkoxycarbonyl- oder Carboxamidgruppe, substituiertes oder unsubstituiertes C₅-C₂₀-Aryl, C₅-C₂₀-Heteroaryl, Arylalkyl, wobei Alkenyle und Alkinyle auch an zwei nebeneinander liegenden C-Atomen des Cyclogrundgerüsts der Verbindung 1 gebunden sein können und die Doppel- bzw. Dreifachbindungen mit dem Ring konjugiert sein können,
X und Y gleich oder verschieden CH₂, NH, O, S oder bilden gemeinsam CH = CH,
h= 1, 2, 3
**dadurch gekennzeichnet,**
**dass** man eine entsprechende Carbonsäure der allgemeinen Formel **2** 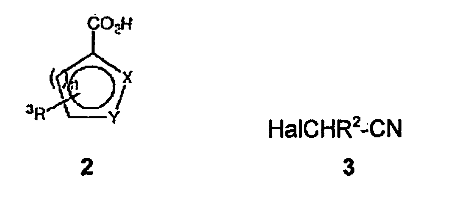 zur Durchführung einer Birch-Reduktion mit Halogenacetonitrilen der allgemeinen Formel **3,** wobei R², R³, X, Y und n die o.g. Bedeutungen besitzen und Hal für Cl oder Br steht,
in flüssigem Ammoniak in Gegenwart von Alkalimetallen umsetzt, anschließend das entstandene Nitril der allgemeinen Formel **4** einer katalytischen Hydrierung unterzieht, wodurch das entsprechende Amin der allgemeinen Formel 5 gebildet wird, 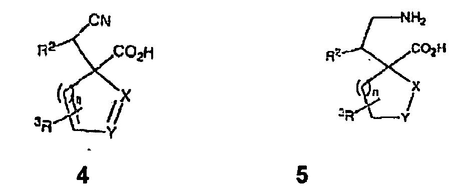 wobei R², R³, X, Y und n die o.g. Bedeutung besitzen,
welches unter Erwärmen zur Zielverbindung 1 dehydratisiert wird.

2. Verfahren nach Anspruch 1, **dadurch gekennzeichnet, dass** als Alkalimetall zur Birch-Reduktion Lithium, Natrium oder Kalium verwendet wird, vorzugsweise Lithium.

3. Verfahren nach Anspruch 1 oder 2, **dadurch gekennzeichnet, dass** n = 1 oder 2, vorzugsweise 2 bedeutet.

4. Verfahren nach einem der Ansprüche 1 bis 3, **dadurch gekennzeichnet, dass** R² Wasserstoff, Halogen, C₁-C₆-Alkyl, Aryl oder Heteroaryl bedeutet.

5. Verfahren nach einem der Ansprüche 1 bis 4, **dadurch gekennzeichnet, dass** R³ einfach oder mehrfach H, C₁ bis C₆ Alkyl oder C₂ bis C₆ Alkenyle und Alkinyle, die auch an zwei nebeneinander liegenden C-Atomen des Cyclogrundgerüsts der Verbindung 1 gebunden sein können und die Doppel- bzw. Dreifachbindungen mit dem Ring konjugiert sein können, sowie Carbonyl-, Carboxyl-, Carboxamid-, Ester- oder Sulfoxylgruppen oder Aryle und Heteroaryle, die ggf. sämtlich substituiert sind, bedeutet.

6. Verfahren nach einem der Ansprüche 1 bis 5, **dadurch gekennzeichnet, dass** die Birch-Reduktion in Gegenwart von Lithium in einem Chloracetonitril erfolgt.

7. Verfahren nach einem der Ansprüche 1 bis 6, **dadurch gekennzeichnet, dass** die zyklische Carbonsäure oder Heterocarbonsäure der allgemeinen Formel 2 ausgewählt ist aus der Gruppe bestehend aus Furan-2-carbonsäure, Pyrrol-2-carbonsäure, Thiophen-2-carbonsäure, Isoxazolcarbonsäure, Isoxthiazolcarbonsäure, 1 H-Pyrazolcarbonsäure, Benzoesäure, Salicylsäure, 3,4,-Dihydroxybenzoesäure, o-Toluylsäure, m-Toluylsäure, p-Toluylsäure, 1-Naphthoesäure, 2-Naphthoesäure, 9-Anthracoesäure, 2-Pyridincarbonsäure, 3-Pyridincarbonsäure, 4-Pyridincarbonsäure, 2-Chinolincarbonsäure, 3-Chinolincarbonsäure und Isochinolin-1-carbonsäure.

8. Verfahren nach einem der Ansprüche 1 bis 7, **dadurch gekennzeichnet**, die zyklische Carbonsäure oder Heterocarbonsäure der allgemeinen Formel 2 ausgewählt ist aus der Gruppe, die die Bildung eines Cyclohexanringes gewährleistet.

9. Verfahren nach einem der Ansprüche 1 bis 8, **dadurch gekennzeichnet, dass** der Katalysator zur katalytischen Hydrierung ausgewählt ist aus der Gruppe umfassend Palladium, Platin und Nickel.

10. Verfahren nach einem der Ansprüche 1 bis 9, **dadurch gekennzeichnet, dass** ein γ-Spirolactam der allgemeinen Formel 1 zur weiteren Synthese von Verbindungen der allgemeinen Formel 1 a verwendet wird, 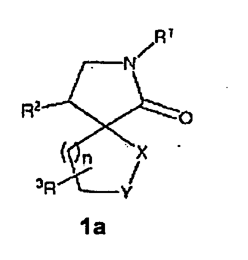 wobei R², R³, X, Y und n die für Verbindung 1 genannten Bedeutungen besitzen und R¹ einen Rest darstellt, der ausgewählt ist aus der Gruppe der für R³ genannten Bedeutungen.

## Claims

1. A method for producing γ-spirolactams of general formula 1 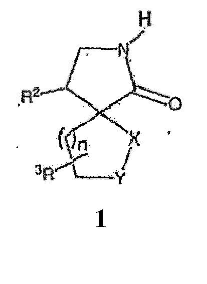 in which
R² is H, substituted or unsubstituted C₁-C₂₀ alkyl, C₂-C₂₀ alkenyl, C₂-C₂₀ alkynyl, C₁-C₂₀ O-alkyl, C₂-C₂₀ O-alkenyl, C₂-C₂₀ O-alkynyl, halogen, SH, NH₂, CN, NO₂, SO₃, a carbonyl, thiocarbonyl, imino, carboxyl, C₁-C₂₀ alkoxycarbonyl or carboxamide group, substituted or unsubstituted C₅-C₂₀ aryl, C₅-C₂₀ heteroaryl, and
R³ is in one or more cases H, substituted or unsubstituted C₁-C₂₀ alkyl, C₂-C₂₀ alkenyl, C₂-C₂₀ alkynyl, C₁-C₂₀ O-alkyl, C₂-C₂₀ O-alkenyl, C₂-C₂₀ O-alkynyl, C₃-C₈ cycloalkyl, C₃-C₈ cycloalkylalkyl, halogen, hydroxyl, SH, NH₂, CN, NO₂, SO₃, a carbonyl, thiocarbonyl, imino, carboxyl, C₁-C₂₀ alkoxycarbonyl or carboxamide group, substituted or unsubstituted C₅-C₂₀ aryl, C₅-C₂₀ heteroaryl, arylalkyl, wherein alkenyls and alkynyls may also be bound to two adjacent C atoms of the cyclo skeleton of compound 1 and the double and triple bonds may be conjugated to the ring,
X and Y are identical or different and are CH₂, NH, O, S or together form CH = CH,
n = 1, 2, 3,
**characterized**
**in that** a corresponding carboxylic acid of general formula 2 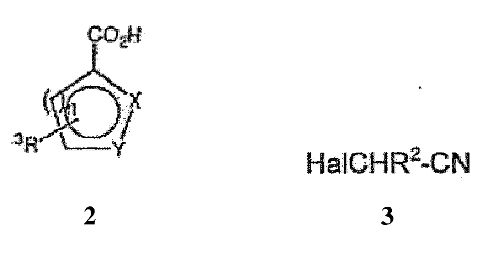 in order to carry out a Birch reduction is reacted with haloacetonitriles of general formula 3, in which R², R³, X, Y and n have the meanings given above and Hal represents Cl or Br, in liquid ammonia and in the presence of alkali metals, then the resulting nitrile of general formula 4 is subjected to catalytic hydrogenation, as a result of which the corresponding amine of general formula 5 is formed, 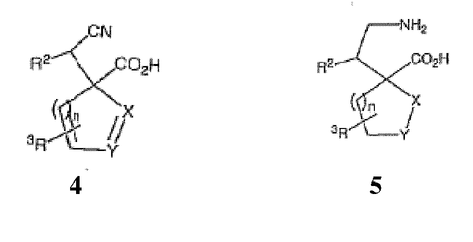 in which R², R³, X, Y and n have the meaning given above,
which is dehydrated by heating to give the target compound 1.

2. The method according to claim 1, **characterized in that** the alkali metal used for the Birch reduction is lithium, sodium or potassium, preferably lithium.

3. The method according to claim 1 or 2, **characterized in that** n = 1 or 2, preferably 2.

4. The method according to any of claims 1 to 3, **characterized in that** R² is hydrogen, halogen, C₁-C₆ alkyl, aryl or heteroaryl.

5. The method according to any of claims 1 to 4, **characterized in that** R³ is in one or more cases H, C₁-C₆ alkyl or C₂-C₆ alkenyls and alkynyls, which may also be bound to two adjacent C atoms of the cyclo skeleton of compound 1 and the double and triple bonds may be conjugated to the ring, as well as carbonyl, carboxyl, carboxamide, ester or sulphoxyl groups or aryls and heteroaryls, all of which are optionally substituted.

6. The method according to any of claims 1 to 5, **characterized in that** the Birch reduction is carried out in the presence of lithium in a chloroacetonitrile.

7. The method according to any of claims 1 to 6, **characterized in that** the cyclic carboxylic acid or heterocarboxylic acid of general formula 2 is selected from the group consisting of furan-2-carboxylic acid, pyrrole-2-carboxylic acid, thiophene-2-carboxylic acid, isoxazolecarboxylic acid, isothiazolecarboxylic acid, 1H-pyrazolecarboxylic acid, benzoic acid, salicylic acid, 3,4-dihydroxybenzoic acid, o-toluic acid, m-toluic acid, p-toluic acid, 1-naphthoic acid, 2-naphthoic acid, 9-anthroic acid, 2-pyridinecarboxylic acid, 3-pyridinecarboxylic acid, 4-pyridinecarboxylic acid, 2-quinolinecarboxylic acid, 3-quinolinecarboxylic acid and isoquinoline-1-carboxylic acid.

8. The method according to any of claims 1 to 7, **characterized in that** the cyclic carboxylic acid or heterocarboxylic acid of general formula 2 is selected from the group which ensures the formation of a cyclohexane ring.

9. The method according to any of claims 1 to 8, **characterized in that** the catalyst for the catalytic hydrogenation is selected from the group comprising palladium, platinum and nickel.

10. The method according to any of claims 1 to 9, **characterized in that** a γ-spirolactam of general formula 1 is used for the further synthesis of compounds of general formula 1a, 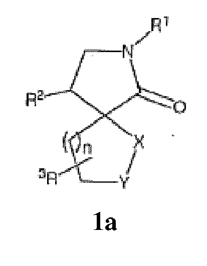 in which R², R³, X, Y and n have the meanings given for compound 1 and R¹ represents a radical selected from the group of meanings given for R³.

## Revendications

1. Procédé de fabrication de spirolactames gamma de formule générale 1 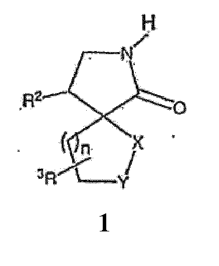 où
R² représente H, un alkyle C₁-C₂₀, un alcényle C₂-C₂₀, un alcynyle C₂-C₂₀, un O-alkyle C₁-C₂₀, un O-alcényle C₂-C₂₀, un O-alcynyle C₂-C₂₀, substitués ou non substitués, un halogène, SH, NH₂, CN, NO₂, SO₃, un groupe carbonyle, thiocarbonyle, imine, carboxyle, alkoxycarbonyle C₁-C₂₀ ou carboxamide, un aryle C₅-C₂₀, un hétéroaryle C₅-C₂₀, substitués ou non substitués, et
R³ représente un ou plusieurs H, un alkyle C₁-C₂₀, un alcényle C₂-C₂₀, un alcynyle C₂-C₂₀, un O-alkyle C₁-C₂₀, un O-alcényle C₂-C₂₀, un O-alcynyle C₂-C₂₀, un cycloalkyle C₃-C₈, un cycloalkylalkyle C₃-C₈, substitués ou non substitués, un halogène, un groupe hydroxyle, SH, NH₂, CN, NO₂, SO₃, un groupe carbonyle, thiocarbonyle, imine-, carboxyle, alkoxycarbonyle C₁-C₂₀ ou carboxamide, un aryle C₅-C₂₀, un hétéroaryle C₅-C₂₀, un arylalkyle, substitués ou non substitués, l'alcényle et l'alcynyle pouvant également être liés à deux atomes de carbone du squelette de base cyclique du composé 1 voisins, et les doubles ou triples liaisons pouvant être conjuguées avec le cycle,
X et Y représentent CH₂, NH, O, S, égaux ou différents, ou forment conjointement CH = CH, n = 1, 2, 3,
**caractérisé en ce qu'**
on fait réagir un acide carboxylique correspondant de formule générale 2 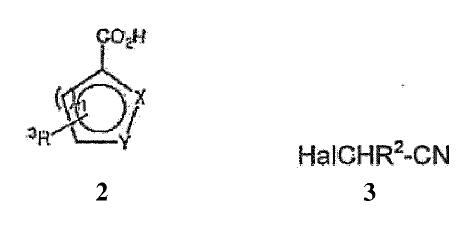 pour déclencher, dans de l'ammoniac liquide en présence de métaux alcalins, une réduction de Birch avec des acétonitriles halogénés de formule générale 3, R², R³, X, Y et n ayant les significations mentionnées ci-dessus et Hal représentant Cl ou Br, et **en ce qu'**on soumet ensuite le nitrile obtenu de formule générale 4 à une hydrogénation catalytique, par laquelle l'amine correspondante de formule générale 5 est formée, 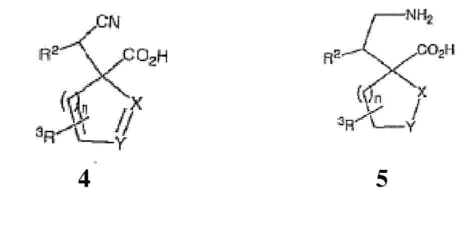 R², R³, X, Y et n possédant la signification mentionnée ci-dessus,
laquelle est déshydratée par chauffage en composé cible 1.

2. Procédé selon la revendication 1, **caractérisé en ce que**, comme métal alcalin pour la réduction de Birch, du lithium, du sodium ou du potassium sont employés, de préférence du lithium.

3. Procédé selon la revendication 1 ou 2, **caractérisée en ce que** n = 1 ou 2, représente de préférence 2.

4. Procédé selon l'une des revendications 1 à 3, **caractérisé en ce que** R² représente l'hydrogène, un halogène, un alkyle C₁-C₆, un aryle ou un hétéroaryle.

5. Procédé selon l'une des revendications 1 à 4, **caractérisé en ce que** R³ représente un ou plusieurs H, alkyle C₁-C₆ ou alcényle et alcynyle C₂-C₆, pouvant également être liés à deux atomes de carbone du squelette de base cyclique du composé 1 voisins et les doubles ou triples liaisons pouvant être conjuguées avec le cycle, ainsi que des groupes carbonyle, carboxyle, carboxamide, ester ou sulfoxyle ou aryle et hétéroaryle, qui, le cas échéant sont tous substitués.

6. Procédé selon l'une des revendications précédentes 1 à 5, **caractérisé en ce que** la réduction de Birch se produit dans un chloracétonitrile en présence de lithium.

7. Procédé selon l'une des revendications 1 à 6, **caractérisé en ce que** l'acide carboxylique cyclique ou l'acide hétéro-carboxylique de formule générale 2 est choisi parmi le groupe se composant d'acide furan-2-carboxylique, d'acide pyrrol-2-carboxylique, d'acide thiophène-2-carboxylique, d'acide isoxazol-carboxylique, d'acide isothiazole-carboxylique, d'acide 1H-pyrazole-carboxylique, d'acide benzoïque, d'acide salicylique, d'acide 3,4-dihydroxybenzoïque, d'acide o-toluylique, d'acide m-toluylique, d'acide p-toluylique, d'acide 1-naphtoïque, d'acide 2-naphtoïque, d'acide 9-anthroïque, d'acide pyridine-carboxylique-2, d'acide pyridine-carboxylique-3, d'acide pyridine-carboxylique-4, d'acide 2-quinoléine-carboxylique, d'acide 3-quinoléine-carboxylique, et d'acide isoquinoléine-1-carboxylique,

8. Procédé selon l'une des revendications 1 à 7, **caractérisé en ce que** l'acide carboxylique cyclique ou l'acide hétéro-carboxylique de formule générale 2 est choisi parmi un groupe garantissant la formation d'un cyclohexane.

9. Procédé selon l'une des revendications 1 à 8, **caractérisé en ce que** le catalyseur pour l'hydrogénation catalytique est choisi parmi un groupe comprenant le palladium, le platine et le nickel.

10. Procédé selon l'une des revendications 1 à 9, **caractérisé en ce qu'**un spirolactame gamma de formule générale 1 est employé pour la synthèse des composés de formule générale 1a, 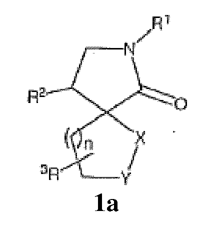 R², R³, X, Y et n possédant les significations mentionnées pour le composé 1 et R^{I} représentant un reste, lequel est choisi parmi le groupe des significations mentionnées pour R³.
